(19) Europäisches Patentamt
European Patent Office
Office européen des brevets

(11) **EP 4 635 619 A1**

(12) **EUROPEAN PATENT APPLICATION**

(43) Date of publication:
**22.10.2025 Bulletin 2025/43**

(21) Application number: **25171197.4**

(22) Date of filing: **17.04.2025**

(51) International Patent Classification (IPC):
**B01J 13/04** (2006.01)          **C08J 9/20** (2006.01)
**C08J 9/22** (2006.01)          **C08L 97/00** (2006.01)

(52) Cooperative Patent Classification (CPC):
**B01J 13/043; C08L 97/005**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **18.04.2024 EP 24171007**
**17.05.2024 EP 24176673**

(71) Applicant: **Nouryon Chemicals International B.V.**
**1101 BZ Amsterdam (NL)**

(72) Inventors:
• **WIJTMANS, Roel**
**1101 BZ Amsterdam (NL)**
• **ANDREASSON, Bo**
**1101 BZ Amsterdam (NL)**
• **SVENSSON, Ted**
**1101 BZ Amsterdam (NL)**
• **NEJSTRÖM, Malin**
**1101 BZ Amsterdam (NL)**

(74) Representative: **Ingrassia, Fisher & Lorenz UK Ltd.**
**Cambridge House**
**Henry Street**
**Bath BA1 1BT (GB)**

(54) **EXPANDED MICROSPHERES**

(57)     The present disclosure relates to expanded microspheres comprising a polymeric shell surrounding a hollow core, wherein the exterior surface of the polymeric shell is free from particulate deposits originating from a solid suspending agent, and processes for preparing said expanded microspheres.

Figure 1.

**Description**

**Technical Field**

The present disclosure relates to expanded microspheres, and a process for their production.

**Background**

Thermally expandable microspheres are known in the art, and are described for example in US3615972, WO 00/37547, WO2007/091960, WO2021/234010, US2020/216631 and US2024/149236. A number of examples are sold under the trade name Expancel®. They can be expanded to form extremely low weight and low density fillers, and find use in applications such as foamed or low density resins, paints and coatings, cements, inks and crack fillers. Consumer products that often contain expandable microspheres include lightweight shoe soles (for example for running shoes), textured coverings such as wallpaper, solar reflective and insulating coatings, food packaging sealants, wine corks, artificial leather, foams for protective helmet liners, automotive weather strips, genuine leather, pre-decos, personal care applications, thermoset materials and block materials, thermoplastic packaging, paints, automotive putties and artificial marble. Most of these applications require the expanded microspheres to be substantially white in colour.

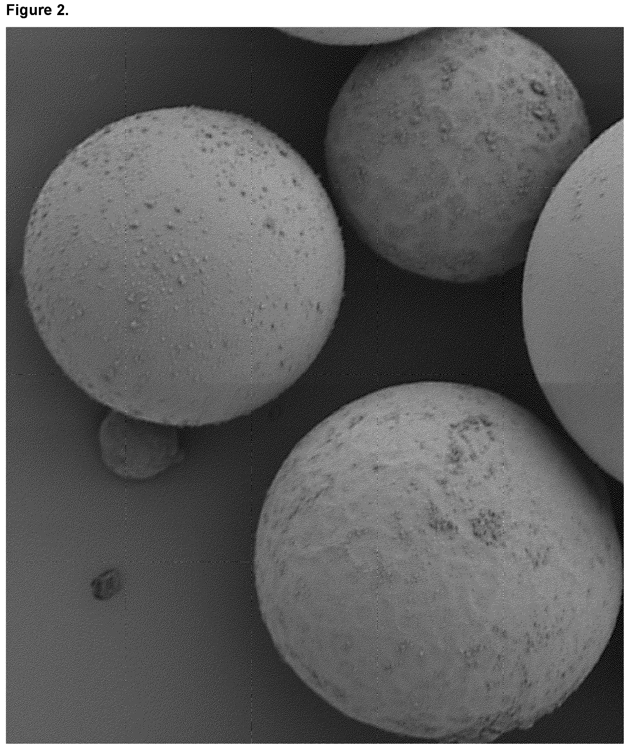

It is generally necessary to add one or more solid suspending agents (e.g., colloidal silica, alumina sol, magnesium hydroxide, etc.) to prevent coalescence during the preparation of the unexpanded microspheres and prevent aggregation of the unexpanded microspheres, particularly in the subsequent expansion process. Whilst the use of solid suspending agents has proven to be - and continues to be - a highly successful approach to solving these agglomeration issues, a drawback with the use of such additives is that they coat the exterior surface of the expanded microspheres (this can be seen in Figure 2, which is an SEM image of a prior art expanded microsphere; the fur-like dark patches/bumps coating the exterior surface of the expanded microspheres are particles of the solid suspending agent). Since, in practice, the solid suspending agents are inorganic agents, disposing the suspending agent on the exterior surface of the microspheres substantially increases the ash content of said microspheres. The inorganic agent also generally adds weight to the microsphere, thus increasing the particle density thereof.

There are many applications, however, wherein a high ash content is undesirable and/or detrimental to the intended application. Expanded microspheres are commonly used as a lightweight filler (e.g., in acrylic coatings), hence reducing the ash content and density of the expanded microspheres has obvious benefits in that respect. Further applications that would benefit from low-ash-content expanded microspheres include, but are not limited to:

- use of the low-ash-content expanded microspheres as a sacrificial template in the production of porous ceramic products (such as filters, high-temperature thermal insulation, membrane reactors, and catalyst supports);
- use of the low-ash-content expanded microspheres in polishing pads for Si wafers, where the absence of inorganic deposits is beneficial;
- use of the low-ash-content expanded microspheres in mortars, such as dry mix mortar.

Also notable in that sense is the pharmaceutical sector, which has shown interest in microspheres as a drug delivery vehicle for many years. Obtaining microspheres with suitable properties for use in such applications, however, has proven to be rather difficult, with most reported methods in the pharmaceutical sector relying on difficult and expensive freeze-thaw or freeze-drying processes (i.e., non-expanded microspheres). Expanded microspheres that contain little to no ash would thus show great potential for use in such low-ash applications, particularly if combined with pharmaceutically acceptable polymers such as poly(lactic acid) (PLA), carboxymethyl cellulose (CMC), and polyvinyl alcohol (PVA).

Accordingly, the primary aim of the present disclosure was to find a way to prepare expanded microspheres without requiring the use of a solid suspending agent.

**Summary**

An unexpected finding was that this objective can be fully realized by directly spray-drying a polymer solution at elevated temperatures. This technical solution was surprisingly effective, as not only did the process allow for the preparation of expanded microspheres without the use of a solid suspending agent, but it was also found to be successful even for polymers that could not be used in the prior art techniques for preparing expanded microspheres, most notably polyvinyl alcohol and carboxymethyl cellulose. Whilst a blowing agent could also be used in the process, it was found that expanded microspheres could be obtained by the process without necessarily requiring the use of a blowing agent. Thus, in a first aspect, the present disclosure relates to a process for preparing expanded microspheres, the process comprising:

    a) obtaining a composition comprising a polymer dissolved in a solvent, wherein the polymer has a glass transition temperature ($T_g$, °C),

b) spray-drying the composition of step a) in a spray dryer, the spray dryer comprising an inlet drying gas having an inlet temperature ($T_{inlet}$, °C), wherein the minimum inlet temperature of the drying gas ($T_{inlet, min}$, °C) is

$$T_{inlet, min} = (T_g*0.75),$$

wherein the maximum inlet temperature of the drying gas ($T_{inlet, max}$, °C) is

$$T_{inlet, max} = (T_g*2.2)$$

and wherein $T_{inlet, max} \geq T_{inlet} \geq T_{inlet, min}$.

For the avoidance of doubt, "$T_g*0.75$" means the glass transition temperature of the polymer ($T_g$, in °C) multiplied by a factor of 0.75. For example, polystyrene with a $T_g$ of 100°C would require a $T_{inlet, min}$ of 75°C and a $T_{inlet, max}$ of 220°C.

It was found that the minimum inlet temperature ($T_{inlet, min}$) could be reduced by including a blowing agent in the composition of step a). When using a blowing agent, it was found that the blowing agent could not have a boiling point that was substantially higher (i.e., >20°C) than the inlet temperature ($T_{inlet}$), otherwise the expansion would fail (i.e., expanded microspheres would not be obtained). Accordingly, in a second aspect, the present disclosure relates to a process for preparing expanded microspheres, the process comprising:

a) obtaining a composition comprising a polymer dissolved in a solvent, wherein the polymer has a glass transition temperature ($T_g$, °C), and wherein the composition further comprises a blowing agent,

b) spray-drying the composition of step a) in a spray dryer, the spray dryer comprising an inlet drying gas having an inlet temperature ($T_{inlet}$, °C), wherein the minimum inlet temperature ($T_{inlet, min}$, °C) of the drying gas is

$$T_{inlet, min} = (T_g*0.60),$$

wherein the maximum inlet temperature of the drying gas ($T_{inlet, max}$, °C) is

$$T_{inlet, max} = (T_g*2.2),$$

wherein $T_{inlet, max} \geq T_{inlet} \geq T_{inlet, min}$, and

wherein the blowing agent boiling point temperature ($T_{BABP}$, °C) is

$$T_{BABP} \leq (T_{inlet} + 20°C).$$

For the avoidance of doubt, "$T_g*0.60$" means the glass transition temperature of the polymer ($T_g$, in °C) multiplied by a factor of 0.60. For example, polystyrene with a $T_g$ of 100°C would require a $T_{inlet, min}$ of 60°C and a $T_{inlet, max}$ of 220°C when a blowing agent is used in step a).

For the avoidance of doubt, $T_{BABP} \leq (T_{inlet} + 20°C)$ means the boiling point of the blowing agent must be less than or equal to the inlet temperature plus 20°C. For example, if the inlet temperature, $T_{inlet}$, is 70°C, then the blowing agent must have a boiling point of ≤90°C.

An added benefit of these processes is that the density of the expanded microspheres can be predictably and controllably tuned to the desired amount by adjusting the inlet temperature, $T_{inlet}$. Moreover, by keeping the inlet temperature between the stated maximum and minimum temperatures (i.e., $T_{inlet, max} \geq T_{inlet} \geq T_{inlet, min}$), the process yields substantially white expanded microspheres that have not agglomerated and which have an ISO brightness value of at least 70 (with the caveat that, for obvious reasons, substantially white microspheres cannot be obtained when using intrinsically non-white polymers, such as lignin).

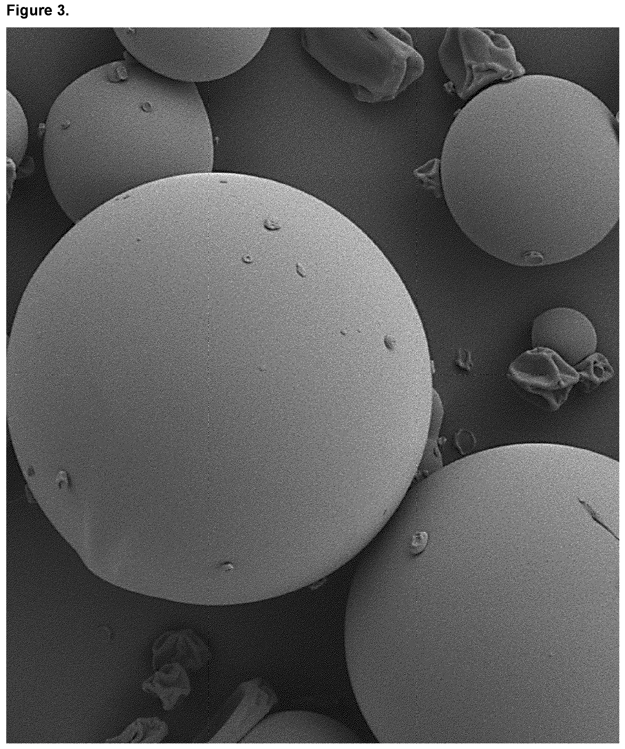

These processes give rise to expanded microspheres without requiring the use of solid suspending agents. Accordingly, in a third aspect, the present disclosure relates to expanded microspheres comprising a polymeric shell surrounding a hollow core, wherein the exterior surface of the polymeric shell is free from particulate deposits originating from a solid suspending agent. As can be seen from Figure 3, these expanded microspheres are of excellent quality and do not contain any solid suspending agent particles on the exterior surface of the polymeric shell. The only apparent "blemishes" on the exterior surface of the polymeric shell are in fact a statistically inevitable small amount of very small microspheres as a result of the Gaussian size distribution (these are less obvious in Figure 2 due to the solid suspending agent coating the exterior surface). The expanded microspheres disclosed herein are therefore particularly suitable for use as low ash, low density filler materials. Accordingly, in a fourth aspect, the present disclosure relates to the use of the expanded microspheres disclosed herein as a filler material, e.g., as a sacrificial template in the production of porous ceramic products (such as filters, high-temperature thermal insulation, membrane reactors, and catalyst supports), in polishing pads for Si wafers, or in mortars, such as dry mix mortar.

As noted above, low ash content microspheres show great promise in the pharmaceutical sector, particularly when combined with a pharmaceutically acceptable polymer. Accordingly, in a fifth aspect, the present disclosure relates to the use of the expanded microspheres disclosed herein as a drug delivery vehicle (i.e., a substance used as a medium for administration of a pharmaceutical, which typically serves to improve the selectivity, effectiveness, and/or safety of administration of a pharmaceutical). Preferably, the expanded microspheres comprise a polymeric shell formed from a pharmaceutically acceptable polymer, preferably a biodegradable pharmaceutically acceptable polymer, such as (but not limited to) homo- and co-polymers of lactic acid, carboxymethylcellulose (CMC), and polyvinyl alcohol (PVA). The expanded microspheres may be used as-is (e.g., the drug molecule [commonly referred to as the active pharmaceutical ingredient] may be dispersed in the solvent of step a) and thus become encapsulated or embedded in the resultant expanded microsphere), or the expanded microspheres may be subjected to further chemical modification so that drug molecules may be covalently attached to the exterior surface of the microspheres (e.g., a PVA expanded microsphere may be further functionalized via chemical reaction with the free alcohol (OH) groups on the polymer backbone). Relevant to the fifth aspect is the PVA and CMC expanded microspheres disclosed herein. To the best of the inventors' knowledge, this is the first disclosure of expanded microspheres having a polyvinyl alcohol polymeric shell or a carboxymethyl cellulose polymeric shell surrounding a hollow core (i.e., single-core expanded microspheres with a polyvinyl alcohol shell or carboxymethyl cellulose shell. For completeness, whilst US3960583 appears to disclose PVA microspheres, it has been found by the present inventors that the PVA microspheres that would have been obtained by the process of US3960583 would have been of such poor quality that they would be unusable from a real-world practical viewpoint; see worked examples 37-38 below). This, combined with the essentially zero wt.% ash content, makes these expanded microspheres a particularly promising candidate for use in the pharmaceutical sector, such as a drug delivery vehicle (e.g., PVA is known to be a biocompatible polymer with low toxicity and excellent biodegradation properties). Accordingly, in a sixth aspect, the present disclosure relates to expanded microspheres comprising a polymeric shell surrounding a hollow core, wherein the polymeric shell comprises or consists of a polymer selected from polyvinyl alcohol or carboxymethyl cellulose, and wherein the expanded microspheres have an ISO brightness value of at least 70 (ISO 2470-1:2016). This concept of drug delivery may be extended beyond the pharmaceutical sector to any technical fields wherein delivery of an active ingredient is desired, such as the agrochemical field (e.g., pesticides, plant nutrients [fertilizers], etc.), the cosmetic field (e.g., cosmetic actives, fragrances, etc.), and the home care field (e.g., antimicrobials, air-fresheners, fabric softeners, etc.). Accordingly, it is contemplated that the expanded microspheres of the present disclosure may further comprise an active ingredient encapsulated in the hollow core and/or embedded in the polymeric shell. Thus, in a seventh aspect, the present disclosure relates to the use of expanded microspheres disclosed herein as an active ingredient delivery vehicle.

## Brief Description of Drawings

Figure 1 illustrates the difference between a hollow (single-core) expanded microsphere according to the present disclosure (A; polymer shell 1 and single core 2) and multi-core microsphere not according to the present disclosure (B; polymer shell 1 and multiple cores 2).

Figure 2 is a SEM image ($10^3$x magnification) of prior art expanded microspheres with solid suspending agent particles on the exterior surface of the microsphere (comparative).

Figure 3 is a SEM image ($10^3$x magnification) of expanded microspheres in accordance with the present disclosure (inventive).

Figure 4 shows microscope images (10x magnification) of expanded microspheres (A) and unexpanded microspheres (B).

## Detailed Description

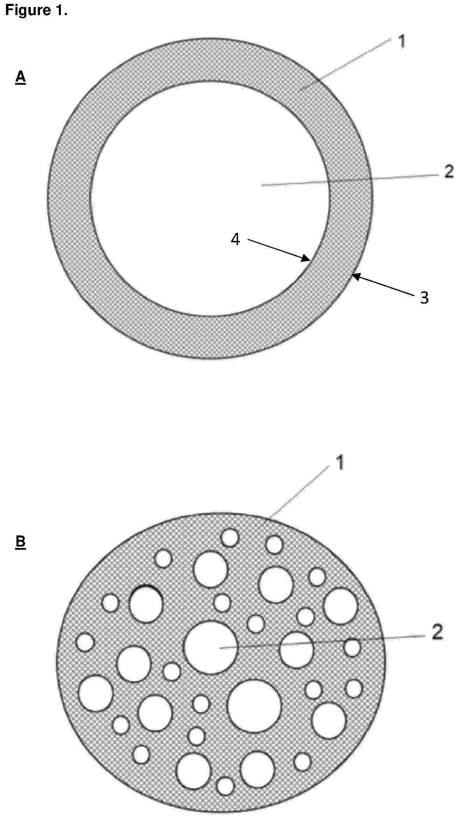

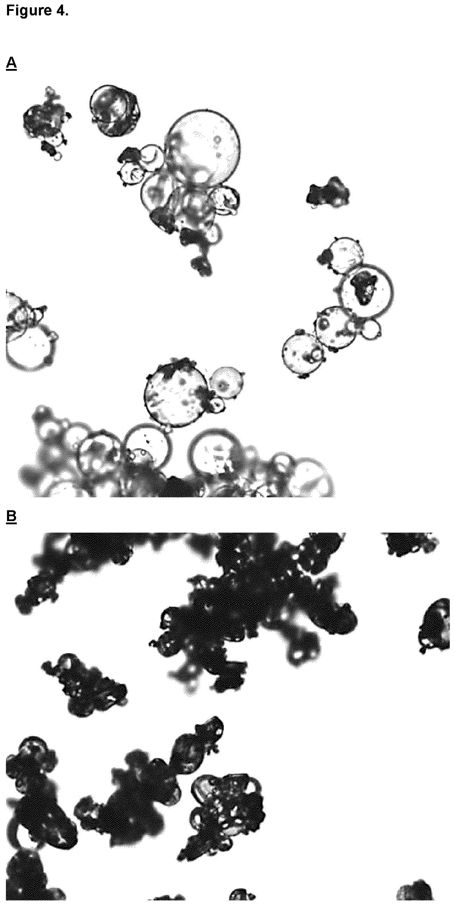

The term "expanded microspheres" has its well-established meaning in this technical field, namely microspheres that have been thermally expanded, yielding low-density, uniform shape, single-core microspheres having a polymeric shell and a single hollow core (as illustrated in Figure 1A and shown in microscope image Figure 4A). For the avoidance of any doubt, the term "expanded microspheres" does not include microspheres that have been prepared by freeze-thaw or freeze-dry processes (which typically yield multi-core porous microspheres as illustrated in Figure 1B, which are like a microspherical foam or sponge).

The term "glass transition temperature (T$_g$)" has its ordinary technical meaning and can be calculated approximately in advance using the Fox equation (according to Fox T. G., Bull. Am. Phys. Soc. 1, p. 123 (1956))

$$\frac{1}{Tg} = \frac{X_A}{Tg_A} + \frac{X_B}{Tg_B} + \cdots + \frac{X_N}{Tg_N}$$

where X$_N$ stands for the mass fraction (wt %/100) of the respective monomer N, and Tg$_N$ is the glass transition temperature, in Kelvin, of the homopolymer of monomer N. Tg values for homopolymers are listed for example in Ullmann's Encyclopedia of Industrial Chemistry, VCH, Weinheim, Vol. A21 (1992), p. 169. The glass transition temperature Tg of the (co-) polymers and/or polymer blends can further be determined experimentally, for example by means of differential scanning calorimetry (DSC) by determining the Tg (Midpoint) of the copolymer according to ASTM D3418-82(1988)e1. DSC is a well-established technique in the technical field for determining Tg, and is the preferred method for determining the Tg of the polymers used in the process of the present disclosure.

As used herein, "ISO brightness value" means the brightness value obtained for expanded microspheres when evaluated using ISO standard ISO 2470-1:2016.

As used herein, "CIELAB values" means the CIELAB whiteness values (L, a, b) obtained for expanded microspheres when evaluated using ISO standard ISO 11475:2017.

As used herein, the term "minimum inlet temperature of the drying gas (T$_{inlet, min}$)" means the minimum temperature of the drying gas at the inlet of the spray dryer that is required to obtain expanded microspheres from the spray-expansion process disclosed herein.

As used herein, the term "maximum inlet temperature of the drying gas (T$_{inlet, max}$)" means the maximum temperature of the drying gas at the inlet of the spray dryer that is required to obtain expanded microspheres from the spray-expansion process disclosed herein.

As used herein, the term "inlet temperature (T$_{inlet}$)" means the temperature of the drying gas at the inlet of the spray dryer.

For the avoidance of any doubt, the term "T$_{inlet, max} \geq$ T$_{inlet} \geq$ T$_{inlet, min}$" means the temperature of the drying gas at the inlet of the spray dryer must be equal to or greater than the calculated minimum temperature of the drying gas required to obtain expanded microspheres from the spray-expansion process disclosed herein and must also be equal to or less than the calculated maximum temperature of the drying gas required to obtain expanded microspheres from the spray-expansion process disclosed herein (i.e., T$_{inlet}$ must be set at a temperature between T$_{inlet, max}$ and T$_{inlet, min}$).

For the avoidance of any doubt, all temperatures referred to herein are in °C.

As used herein, the term "exterior surface of the polymeric shell" has its ordinary meaning, namely the surface of the polymeric shell that is exposed to the surrounding external environment. For the avoidance of any doubt, the surface of the polymeric shell 1 that is the "exterior surface" thereof is indicated by arrow 3 in Figure 1A; arrow 4 in Figure 1A indicates the interior surface of the polymeric shell 1 that is exposed to the hollow core 2.

As used herein, the term "free from particulate deposits originating from a solid suspending agent" has its ordinary meaning, namely that particulate materials originating from a solid suspending agent are not disposed on the exterior surface of the polymeric shell of the expanded microsphere. The presence or absence of such particulate materials on the exterior surface of the polymeric shell can be easily determined by scanning electron microscopy at 10$^3$x magnification (e.g., 5.54 kx magnification; view field 100 μm; SEM HV 2kV), from which particulates originating from a solid suspending agent can be easily identified by their characteristic "fur-like" appearance on the outer surface of the polymeric shell (as seen in Figure 2) which is not observed when the expanded microsphere is free from such particulates (as seen in Figure 3).

The term "solid suspending agents" is a well-known term of the art that is widely used in the technical field of expandable microspheres, e.g., US3615972, EP0486080, WO2007091960, WO2013178561, WO2019043235. In the field of expandable microspheres, the term "solid suspending agents" is synonymous with the term "dispersion stabilizers" and equivalents thereof, e.g., as used in WO 2019124233. For the avoidance of any doubt, "solid suspending agents" as used herein means solid particulate stabilizers as would be used in "Pickering emulsions". Examples of such "solid suspending agents" include salts, oxides and hydroxides of metals like Ca, Mg, Ba, Zn, Ni and Mn (for example one or more of calcium phosphate, calcium pyrophosphate, magnesium pyrophosphate, calcium carbonate, magnesium hydroxide, magnesium oxide, barium sulphate, calcium oxalate, and hydroxides of zinc, nickel or manganese), starch, methyl cellulose, hydroxypropyl methylcellulose, hydroxypropyl methylcellulose, carboxy methylcellulose, gum agar, silica, colloidal clays, oxide and hydroxide of aluminium or iron. Colloidal silica is the predominant solid suspending agent in the technical field of expandable microspheres.

The term "active ingredient" is a well-understood term in the chemical field that is used to denote any ingredient that provides a desired effect. "Active ingredient" includes, but is not limited to, active pharmaceutical ingredients, agrochemical active ingredients (e.g., pesticides, plant nutrients, etc.), cosmetic active ingredients (e.g., perfumes, topical cosmetic agents, sunscreen UV actives, etc.), and home care active ingredients (e.g., antimicrobial agents, antiviral agents, antibacterial agents, air and/or fabric fresheners [e.g., fragrant oils], fabric softeners, etc.).

The expanded microspheres of the present disclosure are obtainable by a spray drying process comprising dissolving a polymer in a suitable solvent (i.e., a solvent in which the polymer will dissolve), and optionally including a blowing agent, and then spraying the thus obtained mixture into a drying equipment to produce the thermally expanded microspheres having a polymeric shell surrounding a hollow core. Thus, a first aspect of the present disclosure relates to a process for preparing expanded microspheres, the process comprising:

a) obtaining a composition comprising a polymer dissolved in a solvent, wherein the polymer has a glass transition temperature ($T_g$),

b) spray-drying the composition of step a) in a spray dryer, the spray dryer comprising an inlet drying gas having an inlet temperature ($T_{inlet}$), wherein the minimum inlet temperature of the drying gas ($T_{inlet,\ min}$) is

$$T_{inlet,\ min} = (T_g * 0.75),$$

wherein the maximum inlet temperature of the drying gas ($T_{inlet,\ max}$, °C) is

$$T_{inlet,\ max} = (T_g * 2.2)$$

and wherein $T_{inlet,\ max} \geq T_{inlet} \geq T_{inlet,\ min}$.

For the avoidance of doubt, $T_g$, $T_{inlet}$, $T_{inlet,\ min}$, and $T_{inlet,\ max}$ are in °C.

In this first aspect, the minimum temperature of the drying gas at the spray-dryer inlet ($T_{inlet,\ min}$) is preferably $T_{inlet,\ min} = (T_g * 0.80)$, more preferably $T_{inlet,\ min} = (T_g * 0.85)$, and most preferably $T_{inlet,\ min} = (T_g * 0.90)$.

In this first aspect, the maximum temperature of the drying gas at the spray-dryer inlet ($T_{inlet,\ max}$) is preferably $T_{inlet,\ max} = (T_g * 2.1)$, more preferably $T_{inlet,\ max} = (T_g * 2)$.

In a second aspect, the process of the present disclosure employs a blowing agent, hence the second aspect relates to a process for preparing expanded microspheres, the process comprising:

a) obtaining a composition comprising a polymer dissolved in a solvent, wherein the polymer has a glass transition temperature ($T_g$), and wherein the composition further comprises a blowing agent,

b) spray-drying the composition of step a) in a spray dryer, the spray dryer comprising an inlet drying gas having an inlet temperature ($T_{inlet}$), wherein the minimum inlet temperature of the drying gas ($T_{inlet,\ min}$) is

$$T_{inlet,\ min} = (T_g * 0.60),$$

wherein the maximum inlet temperature of the drying gas ($T_{inlet,\ max}$, °C) is

$$T_{inlet,\ max} = (T_g * 2.2),$$

wherein $T_{inlet,\ max} \geq T_{inlet} \geq T_{inlet,\ min}$, and

wherein the blowing agent boiling point temperature ($T_{BABP)}$ is

$$T_{BABP} \leq (T_{inlet} + 20°C).$$

In this second aspect, the minimum temperature of the drying gas at the spray-dryer inlet ($T_{inlet,\ min}$) is preferably $T_{inlet,\ min} = (T_g * 0.65)$, more preferably $T_{inlet,\ min} = (T_g * 0.75)$.

In this second aspect, the maximum temperature of the drying gas at the spray-dryer inlet ($T_{inlet,\ max}$) is preferably $T_{inlet,\ max} = (T_g * 2.1)$, more preferably $T_{inlet,\ max} = (T_g * 2)$.

Preferably, $T_{BABP} \leq (T_{inlet} + 10°C)$, more preferably $T_{BABP} \leq T_{inlet}$.

In principle, the spray drying equipment for performing the spray drying process is not limited and any conventional and commercially available spray drying equipment can be used for the spray drying process. A typical spray drying equipment suitable for the process described herein comprises a drying chamber equipped with a nozzle, an inlet for drying gas and an outlet which connects the drying chamber with a cyclone. Through the nozzle which is normally located at the top of the spraying chamber (but may be also located on any other portion of the spray dryer) the liquid to be atomized is sprayed, usually in combination with a spray gas, into the drying chamber. In the drying chamber, the atomized liquid is dried by the drying gas which is fed into the spraying chamber through the inlet for drying gas. The inlet of drying gas may for instance be located besides the nozzle. The atomized liquid dries and forms particles. The thus obtained particles are then fed together with the drying gas through the outlet of the drying chamber which is normally located in the bottom area of the drying chamber into a cyclone. In the cyclone the particles are separated from the drying air. The drying air may be further filtered to remove any residual particles from the drying air.

A suitable spray drying equipment for performing the spray drying process is the Büchi mini spry dryer B-290 which is commercially available from Büchi/Switzerland.

In step a), the order of adding the polymer, the solvent, and the optional blowing agent is not restricted and any order can be chosen.

However, in a preferred embodiment, in the process for producing the expanded microspheres, the polymer is mixed first with the solvent and, optionally, the blowing agent is added to the mixture.

The mixing of the polymer can be carried out at ambient temperature, although temperatures in the range of from 5 to 75°C can be used. Mixing is performed until the polymer has completely dissolved in the solvent.

In embodiments, the mixture of the polymer with the solvent can be left or stirred for a period of time, for example from 1 to 100 hours, or from 2 to 50 hours. This can be at temperatures in the range of from 10 to 95°C, for example at a temperature of from 20 to 90°C.

In a further step, the optional blowing agent is added to the mixture of the polymer and solvent. Also this mixing step can be carried out at ambient temperature, although temperatures in the range of from 5 to 75°C can be used.

After the addition of the blowing agent to the mixture of the polymer and solvent, the thus obtained mixture may be further stirred for a period of time, for example from 1 to 100 hours, or from 2 to 50 hours. Also this can be at temperatures in the range of from 10 to 95°C, for example at a temperature of from 20 to 90°C.

The mixture comprising the polymer, the solvent, and the optional blowing agent is then sprayed into a drying equipment to produce the thermally expanded microspheres as described herein.

The optional spray gas that is sprayed through the nozzle together with the liquid to be atomized is not particularly limited and may be any suitable spray gas known by the skilled person. For instance, the spray gas may be selected from nitrogen, carbon dioxide, (pressurized) air, noble gases, such as argon, etc. Preferably, in the process for producing expanded microspheres as described herein a spray gas is used and more preferably this spray gas is nitrogen.

Also the drying gas is not particularly limited and may be any suitable drying gas known by the skilled person. For instance, also the drying gas may be selected from nitrogen, carbon dioxide, (pressurized) air, noble gases, such as argon, etc. Preferably, the drying gas is nitrogen.

Further process parameters for running the spray drying equipment, such as the spray gas flow, the inlet temperature of the drying gas when entering the drying chamber, the feed rate of the liquid to be atomized and the aspirator speed to circulate the drying gas in the spray drying equipment can be readily chosen by the skilled person.

An unexpected finding was that the process disclosed herein could be used to successfully prepare expanded microspheres using a wide/diverse range of polymer types, from fully synthetic polymers (e.g., polystyrene, polyvinyl alcohol) all the way through to fully bio-derived polymers (e.g., polylactic acid). As such, the polymers are not considered limiting upon the scope of the process and, hence, it is considered that any polymeric component known to the skilled person can be used as a polymer in the process described herein. Polymer blends are also contemplated, i.e., the term "a polymer" as used herein includes a single polymer as well as blends of two or more polymers. Where a polymer blend is used, the $T_g$ used to calculate $T_{inlet, min}$ is the experimentally determined $T_g$ of the blend, not the $T_g$ of each polymer individually. Suitable polymers for use in the present disclosure include, but are not necessarily limited to, one or more polymers selected from the group consisting of polymers obtained by polymerizing one or more vinyl monomers, lignins, polysaccharides, polysaccharide derivatives, polyesters, polyethers, polyacids, polyols, polyalkenes, polyanhydrides, or any combination thereof. In some embodiments, the one or more vinyl monomers are selected from the group consisting of vinylidene chloride, acrylonitrile, methyl methacrylate, methyl acrylate, methacrylonitrile, methacrylic acid, acrylic acid, styrene, vinyl alcohol, vinyl acetate, and combinations thereof. In some embodiments, the lignins are selected from the group consisting of Kraft lignin, lignosulfonates, lignin/hemicellulose blends, and lignosulfonate/hemicellulose blends. In some embodiments, the polysaccharides and polysaccharide derivatives may be selected from the group consisting of cellulose, cellulose derivatives, chitosan, hemicellulose, and alginates. Preferred cellulose derivatives include cellulose esters and cellulose ethers. In some embodiments, the polymer is selected from Kraft lignin, lignosulfonates, lignosulfonate/hemicellulose blends, cellulose acetate, cellulose acetate propionate, carboxymethyl cellulose, homo- or copolymers of styrene, vinyl acetate, lactic acid, glycolic acid, vinyl alcohol, polyacids, acrylic acid, or a combination thereof. In some embodiments, the polymer is a polyvinyl alcohol obtained by hydrolysis of polyvinyl acetate. The degree of hydrolysis is not considered a limiting factor, however in a preferred embodiment the polyvinyl alcohol has a degree of hydrolysis of at least 50%, preferably at least 70%, preferably at least 80%, such as from 50-100%, 70-100%, or 80-100%. Any combination of the aforementioned polymeric components can be used. Such combinations also include copolymers of any of the aforementioned polymeric components.

To facilitate the spray-expansion process disclosed herein, it is preferable for the polymer(s) to have a number average molecular weight (Mn) in the range of from 500 to 700 000, for example in the range of from 1 000 to 500 000, preferably from 2000 to 400 000. In some embodiments, it is in the range of from 1 000 to 100 000, for example from 1 000 to 80 000, or from 2 000 to 50 000. For the avoidance of doubt, Mn is determined by gel permeation chromatography (GPC) using polystyrene standards.

Determining a suitable solvent for dissolving a given polymer may be achieved by routine solvent screening and/or using simple predictive models, such as comparing the Hildebrand solubility parameters ($\delta$) of the polymer and solvent (e.g., polystyrene has a $\delta$ value of 9.1 cal$^{1/2}$ cm$^{-3/2}$, ethyl acetate also has a $\delta$ value of 9.1 cal$^{1/2}$ cm$^{-3/2}$, hence using the Hildebrand solubility parameter it can be predicted that ethyl acetate is likely to be a good solvent for polystyrene, which, in practice, it is). It is well within the routine capabilities of persons skilled in the art to determine a suitable solvent for dissolving a given polymer. It should be understood that the term "solvent" includes aqueous and organic solvents, and mixtures thereof (e.g., water/alcohol mixtures).

The solvent can be water or an organic solvent, such as those having one or more functional groups selected from esters, amides, aldehydes, ketones, alcohols (including glycols) and ethers, for example those having 3 to 12 carbon atoms. Esters, ketones and ethers may, in embodiments, be part of a cyclic structure. Further examples include haloalkanes having from 1 to 6 carbon atoms and halo-carboxylic acids having from 1 to 6 carbon atoms, where the halogen is selected from fluorine, chlorine, bromine and iodine. The solvent can also be a mixture of water and an organic solvent, such as any of the organic solvents as described above.

Examples of organic solvents that can be used include ethyl acetate, methyl formate, ethyl formate, methyl acetate, n-propyl formate, isopropyl formate, n-propyl acetate, isopropyl acetate, isobutyl acetate, n-butyl acetate, n-pentyl formate, isopentyl formate, n-pentyl acetate, isopentyl acetate, ethyl propionate, isobutyl isobutyrate, n-butyl propionate, ethyl 3-ethoxypropionate, 2-ethylhexyl acetate, acetone, methyl ethyl ketone, diethyl ketone, methyl isobutyl ketone, methyl isoamyl ketone, methyl n-amyl ketone, mesityl oxide, acetophenone, cyclohexanone, diethyl phthalate, ethyl lactate, benzyl acetate, butyrolactone, acetyl acetone, methyl cyclohexanone, benzaldehyde, diisobutyl ketone diacetone alcohol, ethylene glycol, glyceryl-$\alpha$-monochlorohydrin, propylene glycol, glycol ethers (for example propylene glycol monomethyl ether, ethylene glycol mono-methyl ether, ethylene glycol mono-ethyl ether, ethylene glycol mono-n-butyl ether, propylene glycol mono-tert-butyl ether, propylene glycol monopropyl ether, propylene glycol monobutyl ether), glycol ether esters (for example ethylene glycol mono-methyl ether acetate, ethylene glycol mono-ethyl ether acetate, ethylene glycol mono-butyl ether acetate, ethylene glycol diacetate), n-propyl alcohol, isopropyl alcohol, n-butanol, sec-butanol, isobutanol, benzyl alcohol, diisopropyl ether, dimethoxymethane, dimethoxyethane, 1,4-dioxane, 1,3-dioxolane, tetrahydrofuran, anisole, phenetole and dimethyl formamide. Other examples of solvents include dimethyl sulfoxide, toluene, xylene, n-methyl-2-pyrrolidone, methylene chloride, chloroform, carbon tetrachloride, trichloroacetic acid, methylene bromide, methylene iodide, trichloroethylene, and tetrachloroethylene. The organic solvent can be a mixture of two or more solvents. If the solvent is a mixture of two or more organic solvents, it is preferred that one solvent thereof is acetone. In embodiments, the acetone is mixed with an alcohol, such as methanol or ethanol.

In some embodiments, the solvent is a mixture of water and an organic solvent. In such embodiments, it is preferred that the mixture contains water in an amount of not more than 50 wt%, such as not more than 40 wt%, not more than 30 wt%, or not more than 20 wt% of water. In such embodiments, it is further preferred that the mixture contains water in an amount of at least 1 wt%, such as at least 2 wt% or at least 5 wt%, the wt% being based on the total weight of the solvent.

In particularly preferred embodiments, the solvent is selected from one or more of water, methanol, ethanol, ethyl acetate, and acetone. More preferred is that the solvent comprises water, methanol, ethanol, ethyl acetate, or acetone. In some particular preferred embodiments, the solvent comprises acetone. In some embodiments, the solvent comprises a mixture of at least two solvents, which are preferably selected from the group consisting of water, methanol, ethanol, ethyl acetate, acetone, and combinations thereof. In some embodiments, the solvent comprises acetone and water, or acetone and ethanol.

Typically, the polymer content in the mixture for spray drying is typically in the range of from 0.1 to 50 wt%. In embodiments, it can be in the range of from 1 to 40 wt%, for example in the range of from 5 to 35 wt%. The wt% are based on the total weight of the mixture for spray drying.

The amount of the optional blowing agent(s) in the mixture for spray drying is typically in the range of from 0.5 to 50 wt%. In embodiments, it can be in the range of from 0.5 to 40 wt%, for example in the range of from 1 to 30 wt%, from 3 to 25 wt%, or even from 5 to 25 wt%.

The amount of the solvent adds up to 100 wt%. Preferably, the amount of organic solvent is at least 30 wt%, more preferably at least 40 wt% and even more preferably at least 50 wt%. The wt% are based on the total weight of the mixture for spray drying.

When a blowing agent is used in the process, the blowing agent is preferably one or more low boiling hydrocarbons or halogenated hydrocarbons, which are liquid at room temperature, but which vaporize on heating.

For the avoidance of doubt, the blowing agent is different to the solvent that is used to dissolve the polymer. In that respect, the one or more blowing agents generally have a boiling point above 25°C at 5.0 bara pressure or above 25°C at 3.0 bara pressure, where "bara" stands for bar-absolute. In embodiments, they have a boiling point above 25°C at atmospheric pressure (1.013 bara). Typically, they have a boiling point of 250°C or less at atmospheric pressure, for example 220°C or less, or 200°C or less. They are preferably inert, and do not react with the polymer. Boiling points at elevated pressures can be calculated using the Clausius Clapeyron equation.

Examples of blowing agents include alcohols, dialkyl ethers, alkanes and halocarbons, e.g. chlorocarbons, fluorocarbons or chlorofluorocarbons. In embodiments, the alcohol comprises a $C_2$ to $C_8$ alcohol, such as a $C_2$ to $C_6$ alcohol or a $C_2$ to $C_4$ alcohol. In embodiments, the dialkyl ether comprises two alkyl groups each selected from $C_2$ to $C_5$ alkyl groups. In embodiments, the alkane is a $C_4$ to $C_{12}$ alkane. In embodiments, the haloalkane is selected from $C_2$ to $C_{10}$ haloalkanes. The haloalkanes can comprise one or more halogen atoms selected from chlorine and fluorine. The alkyl or haloalkyl groups in the alcohols, dialkyl ethers, alkanes and haloalkanes can be linear, branched or cyclic. One or a mixture of one or more blowing agents can be used.

In embodiments, for environmental reasons, the one or more blowing agents are selected from alcohols, alkyl ethers and alkanes, and in further embodiments the one or more blowing agents are selected from alcohols and alkanes, and preferably are alcohols. Haloalkanes are preferably avoided, due to their potential ozone depletion properties, and also due to their generally higher global warming potential.

Examples of suitable blowing agents that can be used include methanol, ethanol, n-propanol, isopropanol, n-butanol, tert-butyl alcohol, n-pentanol, isopentanol, n-hexanol, isohexanol, heptanol, isoheptanol, octanol, isooctanol, n-pentane, isopentane, neopentane, cyclopentane, cyclohexane, n-butane, isobutane, isohexane, neohexane, heptane, isoheptane, octane, isooctane, isodecane, and isododecane. In preferred embodiments, the blowing agent is selected from $C_2$ to $C_8$ alcohols, such as $C_2$ to $C_6$ alcohols or $C_2$ to $C_4$ alcohols. In further preferred embodiments, the blowing agent comprises a blowing agent selected from isooctane, isohexane, isopentane, tert-butyl acetate, butyl acetate, methyl tert-butyl ether, tert-butyl alcohol, and combinations thereof.

It has been found that with the above-described process it is possible to obtain expanded microspheres comprising a polymeric shell surrounding a hollow core, wherein the exterior surface of the polymeric shell is free from particulate deposits originating from a solid suspending agent. In other words, the present disclosure provides expanded microspheres obtainable by a process that does not use a solid suspending agent, wherein the expanded microspheres comprise a polymeric shell surrounding a hollow core. Furthermore, when using polymers that were intrinsically substantially white (i.e., intrinsically non-coloured polymers, such as, but not limited to, PVA, PLA, CMC, cellulose acetate, cellulose acetate propionate, polymethyl methacrylate, polystyrene, etc.) it was found that substantially white expanded microspheres were obtained in the cyclone. In that respect, the obtained expanded microspheres had an ISO brightness value of at least 70 (ISO 2470-1:2016). Preferably, the expanded microspheres have an ISO brightness value of at least 80 (ISO 2470-1:2016). Preferably, the expanded microspheres have a CIELAB whiteness wherein: the "L" value is at least 90; the "a" value is less than ±5, preferably less than ±2; and the "b" value is less than ±5, preferably less than ±2. Accordingly, a preferred embodiment of the expanded microspheres comprises a polymeric shell surrounding a hollow core, wherein the exterior surface of the polymeric shell is free from particulate deposits originating from a solid suspending agent, and wherein the expanded microspheres have an ISO brightness value of at least 70 (ISO 2470-1:2016). Another preferred embodiment of the expanded microspheres comprises a polymeric shell surrounding a hollow core, wherein the exterior surface of the polymeric shell is free from particulate deposits originating from a solid suspending agent, wherein the expanded microspheres have an ISO brightness value of at least 70 (ISO 2470-1:2016), wherein the expanded microspheres have a CIELAB "L" value of at least 90, wherein the expanded microspheres have a CIELAB "a" value of less than 5, and wherein the expanded microspheres have a CIELAB "b" value of less than 5 (ISO 11475:2017)

Preferably, the polymeric shell of the expanded microspheres comprises one or more polymers selected from the group consisting of Kraft lignin, lignosulfonates, lignin/hemicellulose blends, lignosulfonate/hemicellulose blends, cellulose acetate, cellulose acetate propionate, carboxymethyl cellulose, homo- or co-polymers of styrene, homo- or co-polymers of vinyl acetate, homo- or co-polymers of acrylic acid, homo- or co-polymers of methacrylic acid, homo- or co-polymers of methyl acrylate, homo- or co-polymers of methyl methacrylate, homo- or co-polymers of lactic acid, homo- or co-polymers of glycolic acid, homo- or co-polymers of vinyl alcohol, or a combination thereof.

It was found that the expanded microspheres obtained by the process disclosed herein consistently had a particle density of less than 325 g/L. A low particle density is valuable because it will lower the weight in the final application compared to heavy fillers such as glass microspheres of calcium carbonate.

It was also noted that the expanded microspheres consistently had a bulk density of less than 100 g/L. Preferred expanded microspheres of the present disclosure have a bulk density of less than 80 g/L.

The expanded microspheres of the present disclosure thus present an excellent low-density filler material for applications such as, but not limited to, paints and coatings, cultured marble, UBC (underbody coating for automotive), polyester putties, porous ceramics, mining explosives, sealants and adhesives.

The expanded microspheres typically have volume mean particle sizes (diameters), i.e. D(0.5) values, in the range of from 1 to 500 $\mu$m, such as 5 to 400 $\mu$m or, in embodiments, from 10 to 300 $\mu$m or even from 20 to 200 $\mu$m. Volume mean particle sizes may be determined by any suitable means, such as by laser diffraction.

Preferred expanded microspheres of the present disclosure have an ash content of less than 1 wt.%, preferably less than 0.5 wt.%, preferably less than 0.1 wt.%, preferably less than 0.01 wt.%, and most preferably less than 0.001 wt.%, such as 0 wt.%. Determining the ash content is routine in the art, and for the present disclosure may be determined by thermogravimetric analysis of the expanded microspheres.

As already mentioned, the present disclosure also provides for expanded microspheres comprising a polymeric shell surrounding a hollow core, wherein the polymeric shell is formed from polyvinyl alcohol. This, combined with the essentially zero ash content, makes these expanded microspheres a particularly promising candidate for use in the pharmaceutical sector, such as a drug delivery vehicle (PVA is known to be a biocompatible polymer with low toxicity and excellent biodegradation properties). The inventors already have a general proof-of-concept for this application, as initial studies have found that active ingredients can be successfully incorporated in the microspheres by including an active ingredient in the polymer solution of step a) (eucalyptol - a fragrance - was successfully incorporated into microspheres comprising a cellulose acetate polymeric shell). This proof-of-concept finding shows that the expanded microspheres disclosed herein could be used as a delivery vehicle for a wide variety of active ingredients across a wide range of technical fields, such as, but not limited to, the pharmaceutical sector, the agrochemical sector, the cosmetic sector, and the home care sector.

It is noted that various elements of the present disclosure, including but not limited to preferred ranges for the various parameters, can be combined unless they are mutually exclusive.

**Worked Examples**

The present disclosure will be elucidated by the following examples without being limited thereto or thereby.

General Synthesis Method:

A polymer solution was prepared by dissolving the polymer in a suitable solvent by overnight stirring at room temperature with the use of a magnetic stirrer. For the examples that used a blowing agent, the blowing agent was added to the polymer/solvent mixture and stirred overnight at room temperature with the use of a magnetic stirrer. Each component and relative amounts thereof (in wt. %, provided in parentheses) are set out in Table 1.

The thus obtained mixture was then sprayed-dried using a Büchi Mini Spray Dryer B-290. Nitrogen was used as spray gas with a feed rate of 238-397 l/h (depending on the solvent). The feed rate of the mixture to be spray dried was 4-13 ml/min (depending on the solvent). The temperature of the drying gas at the inlet is set out in Table 1 and the aspirator rate was 38 m$^3$/h.

Dried solids were collected from the bottom of the cyclone and analysed.

Microscopic evaluation of the microspheres collected from the bottom of the cyclone was performed using a Leica DM1000 microscope (10x magnification). Expanded microspheres viewed under the microscope are large, uniformly shaped (i.e., essentially perfectly spherical), substantially transparent (bar the polymeric shell) and generally well dispersed

(as shown in Fig. 4A). Non-expanded microspheres viewed under the microscope are essentially the opposite: small, non-uniformly shaped, substantially opaque, and generally clump together (as shown in Fig. 4B; the opacity is a function of the clumping). This qualitative analysis is more than sufficient for determining whether the microspheres collected from the bottom of the cyclone are expanded microspheres. That said, the empirical studies found that expanded microspheres could be distinguished from unexpanded microspheres by the measured particle density - microspheres that were confirmed as being expanded by microscopic evaluation consistently had a measured particle density of less than 325 g/L.

Densities were measured by adding sample up to 50 vol% of a vial of known volume. The weight of the sample was recorded and, in combination with the known volume of the vial, a bulk density can be calculated. Then, an inert medium that does not affect the sample in any way and with a known density (usually isooctane) was added and the vial was filled completely while making sure the mixture was carefully mixed and no air bubbles were present. By measuring the weight of the added inert medium, the particle density of the product can be calculated with the formula below.

$$particle\ density = \frac{sample\ weight}{\left(vial\ volume - \dfrac{inert\ medium\ weight}{inert\ medium\ density}\right)}$$

Table 1.

| Ex. | Polymer (wt. %) | $T_g$ (°C) | Solvent (wt. %) | Blowing Agent (wt. %) | $T_{inlet}$ (°C) | Expanded microspheres? | Particle density (g/L) | Bulk density (g/L) |
|---|---|---|---|---|---|---|---|---|
| 1 | CA (7.8) | 191 | Acetone (92.2) | --- | 120 | No [Unexpanded] | 396 | 83 |
| 2 | CA (7.8) | 191 | Acetone (92.2) | --- | 180 | Yes | 132 | 32 |
| 3 | CA (7.8) | 191 | Acetone (92.2) | --- | 210 | Yes | 105 | 23 |
| | | | | | | | | |
| 4 | CA (7.5) | 191 | Acetone (88.5) | IP (4.0) | 90 | No [Unexpanded] | 330 | 103 |
| 5 | CA (7.5) | 191 | Acetone (88.5) | IP (4.0) | 120 | Yes | 230 | 66 |
| 6 | CA (7.5) | 191 | Acetone (88.5) | IP (4.0) | 150 | Yes | 139 | 38 |
| 7 | CA (7.5) | 191 | Acetone (88.5) | IP (4.0) | 180 | Yes | 96 | 26 |

| Ex. | Polymer (wt. %) | $T_g$ (°C) | Solvent (wt. %) | Blowing Agent (wt. %) | $T_{inlet}$ (°C) | Expanded microspheres? | Particle density (g/L) | Bulk density (g/L) |
|---|---|---|---|---|---|---|---|---|
| 8 | CAP (11.7) | 144 | Acetone/water (88.3) | --- | 40 | No [Unexpanded] | 1258 | 214 |
| 9 | CAP (11.7) | 144 | Acetone/water (88.3) | --- | 60 | No [Unexpanded] | 832 | 203 |
| 10 | CAP (11.7) | 144 | Acetone/water (88.3) | --- | 80 | No [Unexpanded] | 701 | 190 |
| 11 | CAP (11.7) | 144 | Acetone/water (88.3) | --- | 105 | No [Unexpanded] | 449 | 159 |
| 12 | CAP (11.7) | 144 | Acetone/water (88.3) | --- | 140 | Yes | 205 | 56 |
| 13 | CAP (11.7) | 144 | Acetone/water (88.3) | --- | 160 | Yes | 159 | 51 |
| | | | | | | | | |
| 14 | CAP (11.6) | 144 | Acetone (81.4) | IP (7.0) | 120 | Yes | 226 | 70 |
| 15 | CAP (11.6) | 144 | Acetone (81.4) | IP (7.0) | 150 | Yes | 130 | 44 |
| 16 | CAP (11.6) | 144 | Acetone (81.4) | IP (7.0) | 180 | Yes | 74 | 19 |
| | | | | | | | | |
| 17 | PS (5.4) | 100 | Ethyl Acetate (86.5) | IO (8.1) | 70 | No [Unexpanded] | 604 | 97 |
| 18 | PS (5.4) | 100 | Ethyl Acetate (86.5) | IO (8.1) | 90 | Yes | 117 | 17 |
| 19 | PS (5.4) | 100 | Ethyl Acetate (86.5) | IO (8.1) | 110 | Yes | 96 | 12 |
| 20 | PS (5.4) | 100 | Ethyl Acetate (86.5) | IH (8.1) | 90 | Yes | 154 | 11 |
| 21 | PS (5.4) | 100 | Ethyl Acetate (86.5) | IH (8.1) | 110 | Yes | 94 | 14 |
| | | | | | | | | |
| 22 | PLA (23.1) | 45 | Acetone (69.4) | IO (7.4) | 50 | No [Unexpanded] | 495 | 196 |
| 23 | PLA (23.1) | 45 | Acetone (69.4) | IP (7.4) | 50 | Yes | 197 | 64 |
| 24 | PLA (23.1) | 45 | Acetone (69.4) | IP (7.4) | 80 | Yes | 157 | 41 |
| 25 | PLA (23.1) | 45 | Acetone (69.4) | IP/IB (1:1) (7.4) | 50 | Yes | 128 | 46 |
| 26 | PLA | 45 | Acetone | IP/IB (1:1) | 80 | Yes | 136 | 44 |

| Ex. | Polymer (wt. %) | $T_g$ (°C) | Solvent (wt. %) | Blowing Agent (wt. %) | $T_{inlet}$ (°C) | Expanded microspheres? | Particle density (g/L) | Bulk density (g/L) |
|---|---|---|---|---|---|---|---|---|
| | (23.1) | | (69.4) | (7.4) | | | | |
| | | | | | | | | |
| 27 | PVA (7.1) | 85 | Water (88.1) | IP (4.8) | 110 | Yes | 267 | 71 |
| 28 | PVA (7.1) | 85 | Water (88.1) | IP (4.8) | 140 | Yes | 231 | 52 |
| 29 | PVA (7.1) | 85 | Water (88.1) | IP (4.8) | 170 | Yes | 217 | 58 |
| | | | | | | | | |
| 30 | CMC (1.0) | 185 | Water (96.1) | t-BuOH (2.9) | 140 | Yes | 85 | 9 |
| 31 | CMC (1.0) | 185 | Water (96.1) | t-BuOH (2.9) | 170 | Yes | 114 | 13 |
| 32 | CMC (1.0) | 185 | Water (96.1) | t-BuOH (2.9) | 200 | Yes | 198 | 18 |
| | | | | | | | | |
| 33 | PMMA (15.8) | 105 | Acetone (79.0) | IP (5.3) | 110 | Yes | 98 | 26 |
| 34 | PMMA (15.8) | 105 | Acetone (79.0) | IP (5.3) | 140 | Yes | 68 | 8 |
| | | | | | | | | |
| 35 | LS/PVA (4:1) (28.6) | 155 | Water (64.3) | t-BuOH (7.1) | 150 | Yes | 223 | 76 |
| 36 | LS/PVA (4:1) (28.6) | 155 | Water (64.3) | t-BuOH (7.1) | 180 | Yes | 179 | 54 |

*Greyed-out examples in Table 1 are not in accordance with the present disclosure.*

CA = cellulose acetate, Mn 30,000 g/mol

CAP = cellulose acetate propionate, Mn 25,000 g/mol

PS = recycled polystyrene

PLA = polylactic acid, Mn 8,826 g/mol

PVA = polyvinyl alcohol, Mn 13,000-23,000 g/mol, degree of hydrolysis = 87-89%

CMC = carboxymethyl cellulose

PMMA = polymethyl methacrylate, Mn 35,000 g/mol

LS = lignosulfonate

IB = isobutane, b.p. -12°C

IP = isopentane, b.p. 28°C

IO = isooctane, b.p. 99°C

IH = isohexane, b.p. 60°C

t-BuOH = tert-butanol, b.p. 82°C

Acetone/water = 94/6 acetone/water

The generally observed trend was that when a blowing agent was not used, $T_{inlet}$ had to be at least 75% of $T_g$ to ensure that expanded microspheres were obtained from the bottom of the cyclone. In other words, the minimum gas inlet temperature, $T_{inlet, min}$, when a blowing agent is not used was found to be $T_g*0.75$. When $T_{inlet}$ was less than $T_{inlet, min}$, the microspheres obtained from the bottom of the cyclone were found to be dense, small, and non-uniformly shaped unexpanded microspheres that showed a tendency for clumping together (Fig. 4B, which is a microscope image of the unexpanded microspheres obtained from the bottom of the cyclone in Example 8; cf. Fig. 4A, which is a microscope image of the low-density, large, and uniformly shaped [spherical] expanded microspheres obtained from the bottom of the cyclone in Example 13 that were more evenly dispersed, i.e., no significant tendency for clumping).

When a blowing agent was used, it was found that $T_{inlet}$ could be dropped to around 60% of $T_g$ whilst still ensuring that expanded microspheres were obtained from the bottom of the cyclone (i.e., $T_{inlet, min} = T_g*0.60$). The exception to this was Examples 17 and 22, wherein expanded microspheres were not obtained despite the inlet gas temperature, $T_{inlet,}$ being greater than the calculated $T_{inlet, min}$ ($T_g*0.60$). This was attributed to the blowing agent, isooctane, having a boiling point that was substantially higher than $T_{inlet}$ ($T_{BABP}$ 99°C; $T_{inlet}$ 70°C [Ex. 17] and 50°C [Ex. 22]). Upon increasing the temperature (e.g., Ex. 18) or switching to a lower boiling blowing agent (isopentane, Examples 22-23; isopentane+isobutane, Examples 24-25), expanded microspheres were obtained from the bottom of the cyclone under the same spray dryer settings as Examples 17 and 22, respectively.

An unexpected outcome was the wide/diverse range of polymer types that could be successfully employed in the process, from fully synthetic polymers (polystyrene, polyvinyl alcohol, PMMA) to bio-derived polymers (CA, CAP, PLA, CMC). It was also found that blends of polymers could be used successfully (Examples 35-36; 4:1 blend (wt/wt) lignosulfonate : polyvinylalcohol).

A further unexpected outcome was the ability to prepare expanded polyvinyl alcohol microspheres. To the best of the inventors' knowledge, this is the first instance of expanded microspheres having a polyvinyl alcohol polymeric shell surrounding a hollow core (i.e., single-core expanded microspheres with a polyvinyl alcohol shell). This, combined with the essentially zero ash content, makes these expanded microspheres a particularly promising candidate for use in the pharmaceutical sector, such as a drug delivery vehicle (PVA is known to be a biocompatible polymer with low toxicity and excellent biodegradation properties).

Thermal Limit

Whilst attempting to further develop the range of particle and bulk density profiles of the expanded microspheres obtainable by the process of the present disclosure, it was found that there is an upper limit for the inlet temperature of the inlet drying gas. In that respect, at inlet drying gas temperatures above about $2.2*T_g$, the quality of the expanded microsphere product obtained in the cyclone deteriorated rapidly and significantly, and in some instances even jammed/clogged the spray-drying apparatus, yielding no product in the cyclone and rendering the apparatus unusable until it had undergone an intensive cleaning process (which is clearly problematic from a productivity viewpoint - unnecessary apparatus downtime must be avoided). As such, the maximum inlet temperature of the drying gas ($T_{inlet, max}$, °C) was determined to be about $T_g*2.2$, preferably about $T_g*2.1$, more preferably about $T_g*2$. Specific examples of this include repeats of Examples 27-29 (PVA) and Examples 23-24 (PLA) performed at an inlet gas temperature of $>2.2*T_g$, the results of which are provided in Table 2:

*Table 2.*

| Ex. | Polymer (wt. %) | $T_g$ (°C) | Solvent (wt. %) | Blowing Agent (wt. %) | $T_{inlet}$ (°C) | Product Description |
|---|---|---|---|---|---|---|
| 37 | PVA (7.1) | 85 | Water (88.1) | IP (4.8) | 200 | Unusable product - expanded microspheres recovered in the cyclone were exclusively yellow/brown and a significant proportion had agglomerated |
| 38 | PVA (7.1) | 85 | Water (88.1) | IP (4.8) | 220 | Unusable product - expanded microspheres recovered in the cyclone were exclusively yellow/brown and a significant proportion had agglomerated |
| 39 | PLA (23.1) | 45 | Acetone (69.4) | IP (7.4) | 120 | No microspheres recovered in the cyclone, and the apparatus clogged/jammed, requiring extensive cleaning to bring the apparatus back online |
| 40 | PLA (23.1) | 45 | Acetone (69.4) | IP (7.4) | 170 | No microspheres recovered in the cyclone, and the apparatus clogged/jammed, requiring extensive cleaning to bring the apparatus back online |

The products obtained in the cyclone when $T_{inlet, max} \geq T_{inlet} \geq T_{inlet, min}$ were substantially white, low-density expanded microspheres, i.e., excellent quality expanded microspheres that are suitable for the many applications wherein substantially white expanded microspheres are required (caveat: the examples which used intrinsically coloured polymers, namely the lignosulfonates, yielded otherwise excellent microspheres that were not white in colour due to the intrinsic colouring of the polymer, i.e., the colouring is not caused by the process. Those off-white microspheres are useful for other purposes).

Table 3 exemplifies the difference between expanded microspheres according to the present disclosure and expanded microspheres not according to the present disclosure:

*Table 3.*

| Ex. | **Polymer** (wt. %) | $T_g$ (°C) | **Solvent** (wt. %) | **Blowing Agent** (wt. %) | $T_{inlet}$ (°C) | ISO Brightness* | L§ | a§ | b§ |
|---|---|---|---|---|---|---|---|---|---|
| 29 | PVA (7.1) | 85 | Water (88.1) | IP (4.8) | 170 | 85.20 | 94.68 | -0.30 | 1.42 |
| 37 | PVA (7.1) | 85 | Water (88.1) | IP (4.8) | 200 | 44.22 | 83.32 | 1.21 | 20.32 |
| *determined by ISO 2470-1:2016 (ISO Brightness) §determined by ISO 11475:2017 (CIELAB) | | | | | | | | | |

The expanded PVA microspheres of the present disclosure (Ex. 29; $T_{inlet} = T_g$*2) had an ISO brightness and CIELAB values close to almost perfect white, whereas the expanded PVA microspheres not according to the present disclosure (Ex. 37; $T_{inlet} = T_g$*2.35) had an ISO brightness and CIELAB values clearly indicating significant yellowing (the combination of low ISO brightness of 44.22, relatively low L [whiteness] value of 83.32, and positive (+) "b" value of 20.32 is indicative of significant yellowing, which was confirmed by visual inspection of the microspheres).

In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used in the sense of 'including' rather than to mean 'consisting of'. All prior teachings acknowledged above are hereby incorporated by reference. No acknowledgement of any prior published document herein should be taken to be an admission or representation that the teaching thereof was common general knowledge in Europe or elsewhere at the date hereof.

**Claims**

1. A process for preparing expanded microspheres, the process comprising:

a) obtaining a composition comprising a polymer dissolved in a solvent, wherein the polymer has a glass transition temperature ($T_g$, °C),
b) spray-drying the composition of step a) in a spray dryer, the spray dryer comprising an inlet drying gas having an inlet temperature ($T_{inlet}$, °C), wherein a minimum inlet temperature of the drying gas ($T_{inlet, min}$, °C) is

$$T_{inlet, min} = (T_g*0.75),$$

wherein a maximum inlet temperature of the drying gas ($T_{inlet, max}$, °C) is

$$T_{inlet, max} = (T_g*2.2)$$

and wherein $T_{inlet, max} \geq T_{inlet} \geq T_{inlet, min}$.

2. The process of claim 1, wherein the minimum temperature of the drying gas at the inlet ($T_{inlet, min}$) is

$$T_{inlet, min} \geq (T_g*0.90)$$

and/or wherein the maximum inlet temperature of the drying gas ($T_{inlet, max}$, °C) is

$$T_{inlet, max} = (T_g * 2).$$

3. A process for preparing expanded microspheres, the process comprising:

   a) obtaining a composition comprising a polymer dissolved in a solvent, wherein the polymer has a glass transition temperature ($T_g$, °C), and wherein the composition further comprises a blowing agent,
   b) spray-drying the composition of step a) in a spray dryer, the spray dryer comprising an inlet drying gas having an inlet temperature ($T_{inlet}$, °C), wherein a minimum inlet temperature of the drying gas ($T_{inlet, min}$, °C) is

   $$T_{inlet, min} = (T_g * 0.60),$$

   wherein a maximum inlet temperature of the drying gas ($T_{inlet, max}$, °C) is

   $$T_{inlet, max} = (T_g * 2.2)$$

   wherein $T_{inlet, max} \geq T_{inlet} \geq T_{inlet, min}$,
   wherein the blowing agent boiling point temperature ($T_{BABP}$, °C) is

   $$T_{BABP} \leq (T_{inlet} + 20°C).$$

4. The process of claim 3, wherein the minimum temperature of the drying gas at the inlet ($T_{inlet, min}$) is

   $$T_{inlet, min} = (T_g * 0.75)$$

   and/or wherein the maximum inlet temperature of the drying gas ($T_{inlet, max}$, °C) is

   $$T_{inlet, max} = (T_g * 2).$$

5. The process of any one of claims 1-4, wherein the polymer is selected from the group consisting of:

   • polymers obtained by polymerizing one or more vinyl monomers,
   • lignins,
   • polysaccharides,
   • polysaccharide derivatives,
   • polyesters,
   • polyethers,
   • polyacids,
   • polyols,
   • polyalkenes,
   • polyanhydrides,
   • or any combination thereof

6. The process of any one of claims 1-5, wherein the polymer is selected from the group consisting of Kraft lignin, lignosulfonates, lignin/hemicellulose blends, lignosulfonate/hemicellulose blends, cellulose acetate, cellulose acetate propionate, carboxymethyl cellulose, homo- or co-polymers of styrene, homo- or co-polymers of vinyl acetate, homo- or co-polymers of acrylic acid, homo- or co-polymers of methacrylic acid, homo- or co-polymers of methyl acrylate, homo- or co-polymers of methyl methacrylate, homo- or co-polymers of lactic acid, homo- or co-polymers of glycolic acid, homo- or co-polymers of vinyl alcohol, or a combination thereof.

7. The process of any one of claims 1-6, wherein the polymer has a number average molecular weight of about 500 to about 700 000 g/mol (determined by GPC, polystyrene standards).

8. The process of any one of claims 3-7, wherein the blowing agent is one or more hydrocarbons or halogenated hydrocarbons having a boiling point above 25°C at atmospheric pressure.

9. Expanded microspheres comprising a polymeric shell surrounding a hollow core, wherein the exterior surface of the polymeric shell is free from particulate deposits originating from a solid suspending agent, and wherein the expanded microspheres have an ISO brightness value of at least 70 (ISO 2470-1:2016).

10. The expanded microspheres of claim 9, wherein the polymeric shell comprises or consists of a polymer selected from the group consisting of:

- polymers obtained by polymerizing one or more vinyl monomers,
- polysaccharides,
- polysaccharide derivatives,
- polyanhydrides,
- polyesters,
- polyethers,
- polyacids,
- polyols,
- polyalkenes,
- or any combination thereof

11. Expanded microspheres comprising a polymeric shell surrounding a hollow core, wherein the exterior surface of the polymeric shell is free from particulate deposits originating from a solid suspending agent, and wherein the polymeric shell comprises or consists of a polymer selected from lignin or a lignin derivative.

12. The expanded microspheres of claim 11, wherein the lignin derivative is a lignosulfonate.

13. Expanded microspheres comprising a polymeric shell surrounding a hollow core, wherein the polymeric shell comprises or consists of a polymer selected from polyvinyl alcohol or carboxymethyl cellulose, and wherein the expanded microspheres have an ISO brightness value of at least 70 (ISO 2470-1:2016).

14. The expanded microspheres of any one of claims 9-13, further comprising an active ingredient encapsulated in the hollow core and/or embedded in the polymeric shell.

15. The expanded microspheres of any one of claims 9-14, wherein the expanded microspheres have a particle density of less than 325 g/L.

16. The expanded microspheres of any one of claims 9-15, wherein the expanded microspheres have a bulk density of less than 100 g/L.

17. Use of expanded microspheres obtainable by the process of any one of claims 1-8 or according to any one of claims 9-16 as an active ingredient delivery vehicle.

18. Use of expanded microspheres obtainable by the process of any one of claims 1-8 or according to any one of claims 9-16 as a sacrificial template in the production of porous ceramic products, or in polishing pads for Si wafers, or in mortars, such as dry mix mortar.

**Figure 1.**

Figure 2.

**Figure 3.**

**Figure 4.**

<u>A</u>

<u>B</u>

Europäisches
Patentamt

European
Patent Office

Office européen
des brevets

## EUROPEAN SEARCH REPORT

**Application Number**

EP 25 17 1197

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2022/174947 A1 (NOURYON CHEMICALS INT BV [NL]) 25 August 2022 (2022-08-25) * [0061], [0072]; example 1; table 1 * | 1-8 | INV.<br>B01J13/04<br>C08J9/20<br>C08J9/22<br>C08L97/00 |
| A | WO 2021/234010 A1 (NOURYON CHEMICALS INT BV [NL]) 25 November 2021 (2021-11-25) * the whole document * | 1-8 | |
| A,D | US 2020/216631 A1 (NORDIN OVE [SE] ET AL) 9 July 2020 (2020-07-09) * the whole document * | 1-18 | |
| A | US 3 960 583 A (NETTING DAVID I ET AL) 1 June 1976 (1976-06-01) * example 1 * | 13 | |
| X | EP 1 981 631 B1 (AKZO NOBEL NV [NL]; AKZO NOBEL PULP AND PERFORMANCE CHEMICALS AB [SE]) 30 September 2015 (2015-09-30) * [0044], [0067], [0073]; example 6; table 4 * | 9,10, 14-17 | |
| X | KIM ET AL: "A simple pressing route to closed-cell microcellular ceramics", SCRIPTA MATERIALIA, ELSEVIER, AMSTERDAM, NL, vol. 53, no. 8, 1 October 2005 (2005-10-01), pages 921-925, XP005001053, ISSN: 1359-6462, DOI: 10.1016/J.SCRIPTAMAT.2005.06.032 * paragraph [0002] - paragraph [0002]; figures 1-2; example 1 * | 11,12, 14-16 | TECHNICAL FIELDS SEARCHED (IPC)<br><br>B01J<br>C08J<br>C09J<br>C08L |
| X | CN 109 316 461 A (UNIV SOUTH CHINA TECH) 12 February 2019 (2019-02-12) * In the abstract, under points 2, 3; table 1 and figures 2-3: * | 17,18 | |

The present search report has been drawn up for all claims

| Place of search | Date of completion of the search | Examiner |
|---|---|---|
| The Hague | 28 August 2025 | Mc Donnell, Shane |

EPO FORM 1503 03.82 (P04C01)

## ANNEX TO THE EUROPEAN SEARCH REPORT
## ON EUROPEAN PATENT APPLICATION NO.

EP 25 17 1197

This annex lists the patent family members relating to the patent documents cited in the above-mentioned European search report.
The members are as contained in the European Patent Office EDP file on
The European Patent Office is in no way liable for these particulars which are merely given for the purpose of information.

28-08-2025

| Patent document cited in search report | Publication date | Patent family member(s) | Publication date |
|---|---|---|---|
| WO 2022174947 A1 | 25-08-2022 | BR 112023016665 A2 | 14-11-2023 |
| | | CN 116867566 A | 10-10-2023 |
| | | EP 4294557 A1 | 27-12-2023 |
| | | JP 2024509388 A | 01-03-2024 |
| | | KR 20230145463 A | 17-10-2023 |
| | | US 2024149236 A1 | 09-05-2024 |
| | | WO 2022174947 A1 | 25-08-2022 |
| WO 2021234010 A1 | 25-11-2021 | BR 112022021299 A2 | 06-12-2022 |
| | | CN 115666775 A | 31-01-2023 |
| | | EP 4153347 A1 | 29-03-2023 |
| | | JP 7689985 B2 | 09-06-2025 |
| | | JP 2023525897 A | 19-06-2023 |
| | | KR 20230002769 A | 05-01-2023 |
| | | US 2023220177 A1 | 13-07-2023 |
| | | WO 2021234010 A1 | 25-11-2021 |
| US 2020216631 A1 | 09-07-2020 | BR 112020004306 A2 | 08-09-2020 |
| | | CN 111194239 A | 22-05-2020 |
| | | EP 3678768 A1 | 15-07-2020 |
| | | JP 6817496 B2 | 20-01-2021 |
| | | JP 2020529316 A | 08-10-2020 |
| | | KR 20200038540 A | 13-04-2020 |
| | | US 2020216631 A1 | 09-07-2020 |
| | | US 2022220274 A1 | 14-07-2022 |
| | | WO 2019043235 A1 | 07-03-2019 |
| US 3960583 A | 01-06-1976 | NONE | |
| EP 1981631 B1 | 30-09-2015 | BR PI0707621 A2 | 10-05-2011 |
| | | CN 101378830 A | 04-03-2009 |
| | | EP 1981631 A1 | 22-10-2008 |
| | | ES 2556169 T3 | 13-01-2016 |
| | | JP 5438324 B2 | 12-03-2014 |
| | | JP 2009526118 A | 16-07-2009 |
| | | KR 20080098051 A | 06-11-2008 |
| | | TW 200734043 A | 16-09-2007 |
| | | UA 92207 C2 | 11-10-2010 |
| | | WO 2007091961 A1 | 16-08-2007 |
| | | ZA 200807759 B | 29-07-2009 |
| CN 109316461 A | 12-02-2019 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 3615972 A
- WO 0037547 A
- WO 2007091960 A
- WO 2021234010 A
- US 2020216631 A
- US 2024149236 A
- US 3960583 A
- EP 0486080 A
- WO 2013178561 A
- WO 2019043235 A
- WO 2019124233 A

### Non-patent literature cited in the description

- **FOX T. G.** *Bull. Am. Phys. Soc.*, 1956, vol. 1, 123
- Ullmann's Encyclopedia of Industrial Chemistry. VCH, 1992, vol. A21, 169